# FASCICULE DE BREVET EUROPEEN

(11) **EP 1 752 443 B1**
(45) Date de publication et mention de la délivrance du brevet: **11.07.2012**
(21) Numéro de dépôt: 06291251.4
(22) Date de dépôt: 02.08.2006
(51) Int. Cl.: C07C 233/18, C07C 231/24, A61K 31/165, A61P 25/00, A61P 37/00, A61P 15/08, A61P 9/00, A61P 3/00

(54) **Nouvelle forme cristalline V de l'agomelatine, son procede de preparation et les compositions pharmaceutiques qui la contiennent**
Neue kristalline Form V von Agometalin, Verfahren zur deren Herstellung sowie diese enthaltende pharmazeutische Zusammensetzungen
New crystalline form V of agometaline, the process for its preparation and the pharmaceutical compositions containing it

(30) Priorité: 03.08.2005 FR 0508278
(43) Date de publication de la demande: 14.02.2007
(60) Demande divisionnaire: 10011469.3
(73) Titulaire: Les Laboratoires Servier, 92415 Courbevoie Cedex (FR)
(72) Inventeur: Coquerel, Gérard, 76520 Boos (FR); Linol, Julie, 76000 Rouen (FR); Souvie, Jean-Claude, 76600 Le Havre (FR)

(56) Documents cités:
- EP-A- 0 447 285
- EP-A- 1 564 202
- TINANT B ET AL: "N-[2-(7-METHOXY-1-NAPHTHYL)ETHYL]ACETAMID E, A POTENT MELATONIN ANALOG" ACTA CRYSTALLOGRAPHICA SECTION C. CRYSTAL STRUCTURE COMMUNICATIONS, MUNKSGAARD, COPENHAGEN, DK, vol. C50, no. 6, 1994, pages 907-910, XP009047983 ISSN: 0108-2701
- DEPREUX P ET AL: "SYNTHESIS AND STRUCTURE-ACTIVITY RELATIONSHIPS OF NOVEL NAPHTHALENIC AND BIOISOSTERIC AMIDIC DERIVATIVES AS MELATONIN RECEPTOR LIGANDS" JOURNAL OF MEDICINAL CHEMISTRY, AMERICAN CHEMICAL SOCIETY. WASHINGTON, US, vol. 37, no. 20, 30 septembre 1994 (1994-09-30), pages 3231-3239, XP002016146 ISSN: 0022-2623
- CHILMAN-BLAIR K ET AL: "AGOMELATINE. ANTIDEPRESSANT TREATMENT OF BIPOLAR DISORDER MELATONIN AGONUST/5-HT20 ANTAGONIST" DRUGS OF THE FUTURE, BARCELONA, ES, vol. 28, no. 1, janvier 2003 (2003-01), pages 7-13, XP009036058 ISSN: 0377-8282

## Description

La présente invention concerne une nouvelle forme cristalline V de l'agomélatine ou N-[2-(7-méthoxy-1-naphtyl)éthyl]acétamide de formule (I) : 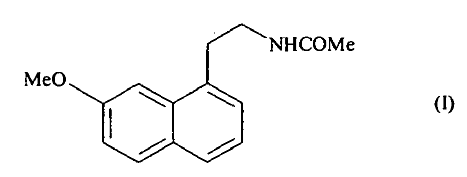 son procédé de préparation ainsi que les compositions pharmaceutiques qui la contiennent.

L'agomélatine ou *N*-[2-(7-méthoxy-1-naphtyl)éthyl]acétamide possède des propriétés pharmacologiques intéressantes.

Il présente en effet la double particularité d'être d'une part agoniste sur les récepteurs du système mélatoninergique et d'autre part antagoniste du récepteur 5-HT_{2C}. Ces propriétés lui confère une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans le brevet européen EP 0 447 285.

Compte tenu de l'intérêt pharmaceutique de ce composé, il était primordial de l'obtenir avec une excellente pureté, et notamment sous une forme parfaitement reproductible, présentant des caractéristiques intéressantes de dissolution et de facilité de formulation permettant son stockage prolongé sans conditions particulières de température, de lumière, d'humidité ou de taux d'oxygène.

Le brevet EP 0 447 285 décrit l'accès en huit étapes à l'agomélatine à partir de la 7-méthoxy-1-tétralone. Cependant, ce document ne précise pas les conditions d'obtention de l'agomélatine sous une forme présentant ces caractéristiques de manière reproductible.

La demanderesse a présentement mis au point un procédé d'obtention de l'agomélatine sous une forme cristalline bien définie, parfaitement reproductible et présentant de ce fait des caractéristiques intéressantes de dissolution et de facilité de formulation.

Plus spécifiquement, la présente invention concerne la forme cristalline V du composé de formule (I), caractérisée par le diagramme de diffraction X sur poudre suivant, mesuré sur un diffractomètre Siemens D5005 (anticathode de cuivre) et exprimé en termes de distance inter-réticulaire d, d'angle de Bragg 2 thêta, et d'intensité relative (exprimée en pourcentage par rapport à la raie la plus intense) :

| **2-Theta (°)** **exp.** | **d (Å)** **exp.** | **Intensité** **(%)** |
|---|---|---|
| 9,84 | 8,979 | 17 |
| 12,40 | 7,134 | 15 |
| 13,31 | 6,646 | 19 |
| 15,14 | 5,848 | 18 |
| 15,98 | 5,543 | 18 |
| 16,62 | 5,329 | 19 |
| 17,95 | 4,939 | 100 |
| 18,88 | 4,697 | 65 |
| 20,49 | 4,332 | 24 |
| 20,99 | 4,228 | 34 |
| 23,07 | 3,852 | 39 |
| 23,44 | 3,792 | 36 |
| 24,28 | 3,663 | 58 |
| 25,10 | 3,545 | 19 |
| 26,02 | 3,422 | 15 |
| 26,82 | 3,322 | 19 |
| 27,51 | 3,239 | 16 |

L'invention s'étend également au procédé de préparation de la forme cristalline V du composé de formule (I), caractérisé en ce que l'on soumet l'agomélatine à un broyage mécanique dit « de haute énergie ».

Dans le procédé de cristallisation selon l'invention, on peut utiliser le composé de formule (I) obtenu par n'importe quel procédé.

L'invention s'étend également à un autre procédé de préparation de la forme cristalline V du composé de formule (I), caractérisé en ce que l'on chauffe l'agomélatine jusqu'à fusion complète puis on le place à température ambiante et simultanément on ajoute une très petite quantité de la forme cristalline V du composé de formule (I) fraîchement préparée, puis on laisse ensuite refroidir jusqu'à cristallisation complète.

De préférence, dans ce second procédé de cristallisation selon l'invention, l'agomélatine sera fondu à 110°C.

La quantité de forme cristalline V ajoutée dans ce second procédé de cristallisation selon l'invention sera comprise de préférence entre 1/100 et 1/50 du poids d'agomélatine.

Dans ce second procédé de cristallisation selon l'invention, on peut utiliser le composé de formule (I) obtenu par n'importe quel procédé.

L'obtention de cette forme cristalline a pour avantage de permettre la préparation de formulations pharmaceutiques ayant une composition constante et reproductible, présentant des caractéristiques de dissolution particulièrement intéressantes, ce qui est particulièrement avantageux lorsque ces formulations sont destinées à l'administration orale.

L'étude pharmacologique de la forme V ainsi obtenue a montré une importante activité sur le système nerveux central ainsi que sur la microcirculation qui permet d'établir son utilité dans le traitement du stress, des troubles du sommeil, de l'anxiété, de la dépression majeure, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, de la douleur, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, la forme V de l'agomélatine peut être utilisée dans les dysfonctionnements sexuels, qu'elle possède des propriétés d'inhibiteurs de l'ovulation, d'immunomodulateurs et qu'elle est susceptible d'être utilisée dans le traitement des cancers.

La forme cristalline V de l'agomélatine sera utilisée de préférence dans les traitements de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'invention s'étend aussi aux compositions pharmaceutiques renfermant comme principe actif la forme cristalline V du composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les granulés, les comprimés sublinguaux, les gélules, les tablettes, les suppositoires, les crèmes, les pommades, les gels dermiques, les préparations injectables, les suspensions buvables et les pâtes à mâcher.

La posologie utile est adaptable selon la nature et la sévérité de l'affection, la voie d'administration ainsi que l'âge et le poids du patient. Cette posologie varie de 0,1 mg à 1 g par jour en une ou plusieurs prises.

Les exemples ci-dessous illustrent l'invention, mais ne la limitent en aucune façon.

### Exemple 1 : Forme cristalline V du N-[2-(7-Méthoxy-1-naphtyl)éthyl]acétamide

100 g de *N*-[2-(7-Méthoxy-1-naphtyl)éthyl]acétamide sont placés dans le broyeur mécanique de type vario-planetary mill pendant environ 6 heures et le solide obtenu est caractérisé par le diagramme de diffraction X sur poudre suivant, mesuré sur un diffractomètre Siemens D5005 (anticathode de cuivre) et exprimé en termes de distance inter-réticulaire d, d'angle de Bragg 2 thêta, et d'intensité relative (exprimée en pourcentage par rapport à la raie la plus intense) :

| **2-Theta (°)** **exp.** | **d (Å)** **exp.** | **Intensité** **(%)** |
|---|---|---|
| 9,84 | 8,979 | 17 |
| 12,40 | 7,134 | 15 |
| 13,31 | 6,646 | 19 |
| 15,14 | 5,848 | 18 |
| 15,98 | 5,543 | 18 |
| 16,62 | 5,329 | 19 |
| 17,95 | 4,939 | 100 |
| 18,88 | 4,697 | 65 |
| 20,49 | 4,332 | 24 |
| 20,99 | 4,228 | 34 |
| 23,07 | 3,852 | 39 |
| 23,44 | 3,792 | 36 |
| 24,28 | 3,663 | 58 |
| 25,10 | 3,545 | 19 |
| 26,02 | 3,422 | 15 |
| 26,82 | 3,322 | 19 |
| 27,51 | 3,239 | 16 |

### Exempte 2 : Forme cristalline V du N-[2-(7-Méthoxy-1-naphtyl)éthyl]acétamide

4 g de *N*-[2-(7-Méthoxy-1-naphtyl)éthyl]acétamide sont placés dans une étuve ventilée à 110°C. Après une heure à 110°C, le produit est mis à température ambiante et ensemencé avec 0,05 g de forme cristalline V du *N*-[2-(7-Méthoxy-1-naphtyl)éthyl]acétamide structuralement pure obtenue par broyage haute énergie. Au bout de 5 minutes, la cristallisation est totale et le solide obtenu est caractérisé par le diagramme de diffraction X sur poudre suivant, mesuré sur un diffractomètre Siemens D5005 (anticathode de cuivre) et exprimé en termes de distance inter-réticulaire d, d'angle de Bragg 2 thêta, et d'intensité relative (exprimée en pourcentage par rapport à la raie la plus intense) :

| **2-Theta (°)** **exp.** | **d (Å)** **exp.** | **Intensité** **(%)** |
|---|---|---|
| 9,84 | 8,979 | 17 |
| 12,40 | 7,134 | 15 |
| 13,31 | 6,646 | 19 |
| 15,14 | 5,848 | 18 |
| 15,98 | 5,543 | 18 |
| 16,62 | 5,329 | 19 |
| 17,95 | 4,939 | 100 |
| 18,88 | 4,697 | 65 |
| 20,49 | 4,332 | 24 |
| 20,99 | 4,228 | 34 |
| 23,07 | 3,852 | 39 |
| 23,44 | 3,792 | 36 |
| 24,28 | 3,663 | 58 |
| 25,10 | 3,545 | 19 |
| 26,02 | 3,422 | 15 |
| 26,82 | 3,322 | 19 |
| 27,51 | 3,239 | 16 |

### Exemple 3 : Composition pharmaceutique

Formule de préparation pour 1000 comprimés doses à 25 mg :

| | |
|---|---|
| Composé de l'Exemple 1 ou 2 | 25 g |
| Lactose monohydrate | 62 g |
| Stéarate de Magnésium | 1,3 g |
| Amidon de maïs | 26 g |
| Maltodextrines | 9 g |
| Silice colloïdale anhydre | 0,3 g |
| Amidon de maïs prégélatinisé type A | 4 g |
| Acide stéarique | 2,6 g |

### Exemple 4 : Composition pharmaceutique

Formule de préparation pour 1000 comprimés doses à 25 mg :

| | |
|---|---|
| Composé de l'Exemple 1 ou 2 | 25 g |
| Lactose monohydrate | 62 g |
| Stéarate de Magnésium | 1,3 g |
| Povidone | 9 g |
| Silice colloïdale anhydre | 0,3 g |
| Cellulose sodium glycolate | 30 g |
| Acide stéarique | 2,6 g |

## Revendications

1. Forme cristalline V de l'agomélatine de formule (I) : 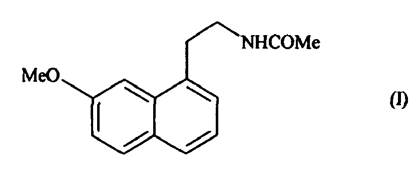 **caractérisée par** le diagramme de diffraction X sur poudre suivant, mesuré sur un diffractomètre Siemens D5005 (anticathode de cuivre) et exprimé en termes de distance inter-réticulaire d, d'angle de Bragg 2 thêta, et d'intensité relative (exprimée en pourcentage par rapport à la raie la plus intense) :
| **2-Theta (°)** **exp.** | **d (Å)** **exp.** | **Intensité** **(%)** |
|---|---|---|
| 9,84 | 8,979 | 17 |
| 12,40 | 7,134 | 15 |
| 13,31 | 6,646 | 19 |
| 15,14 | 5,848 | 18 |
| 15,98 | 5,543 | 18 |
| 16,62 | 5,329 | 19 |
| 17,95 | 4,939 | 100 |
| 18,88 | 4,697 | 65 |
| 20,49 | 4,332 | 24 |
| 20,99 | 4,228 | 34 |
| 23,07 | 3,852 | 39 |
| 23,44 | 3,792 | 36 |
| 24,28 | 3,663 | 58 |
| 25,10 | 3,545 | 19 |
| 26,02 | 3,422 | 15 |
| 26,82 | 3,322 | 19 |
| 27,51 | 3,239 | 16 |

2. Procédé de préparation de la forme cristalline V du composé de formule (I) selon la revendication 1, **caractérisé en ce que** l'on l'on soumet l'agomélatine à un broyage mécanique dans un broyeur de haute énergie

3. Procédé de préparation de la forme cristalline V du composé de formule (I) selon la revendication 1, **caractérisé en ce que** l'on chauffe l'agomélatine jusqu'à fusion complète puis on le place à température ambiante et simultanément on ajoute une très petite quantité de la forme cristalline V du composé de formule (I) fraîchement préparée, puis on laisse ensuite refroidir jusqu'à cristallisation complète.

4. Compositions pharmaceutiques contenant comme principe actif la forme cristalline V de l'agomélatine selon la revendication 1, en combinaison avec un ou plusieurs véhicules inertes, non toxiques et pharmaceutiquement acceptables.

5. Compositions pharmaceutiques selon la revendication 4 pour traiter les troubles du système mélatoninergique.

6. Compositions pharmaceutiques selon la revendication 4 pour le traitement des troubles du sommeil, du stress, de l'anxiété, des dépressions saisonnières ou de la dépression majeure, des pathologies cardiovasculaires, des pathologies du système digestif, des insomnies et fatigues dues aux décalages horaires, de la schizophrénie, des attaques de paniques, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, des troubles de la circulation cérébrale, ainsi que dans les dysfonctionnements sexuels, en tant qu'inhibiteurs de l'ovulation, d'immunomodulateurs et dans le traitement des cancers.

## Claims

1. Crystal form V of agomelatine of formula (I): 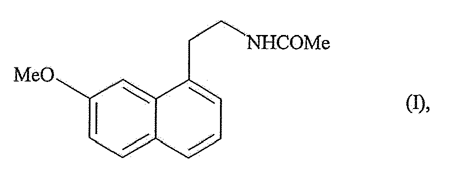 **characterised by** the following X-ray powder diffraction diagram, measured using a Siemens D5005 diffractometer (copper anticathode) and expressed in terms of interplanar spacing d, Bragg's angle 2 theta, and relative intensity (expressed as a percentage in relation to the most intense line):
| 2-theta (°) exp. | d (Å) exp. | Intensity (%) |
|---|---|---|
| 9.84 | 8.979 | 17 |
| 12.40 | 7.134 | 15 |
| 13.31 | 6.646 | 19 |
| 15.14 | 5.848 | 18 |
| 15.98 | 5.543 | 18 |
| 16.62 | 5.329 | 19 |
| 17.95 | 4.939 | 100 |
| 18.88 | 4.697 | 65 |
| 20.49 | 4.332 | 24 |
| 20.99 | 4.228 | 34 |
| 23.07 | 3.852 | 39 |
| 23.44 | 3.792 | 36 |
| 24.28 | 3.663 | 58 |
| 25.10 | 3.545 | 19 |
| 26.02 | 3.422 | 15 |
| 26.82 | 3.322 | 19 |
| 27.51 | 3.239 | 16 |

2. Process for the preparation of the crystal form V of the compound of formula (I) according to claim 1, **characterised in that** agomelatine is subjected to mechanical milling in a high-energy mill.

3. Process for the preparation of the crystal form V of the compound of formula (I) according to claim 1, **characterised in that** agomelatine is heated until melting is complete and then is placed at ambient temperature and, simultaneously, there is added a very small amount of the freshly prepared crystal form V of the compound of formula (I), and it is then allowed to cool until crystallisation is complete.

4. Pharmaceutical compositions comprising, as active ingredient, the crystal form V of agomelatine according to claim 1, in combination with one or more pharmaceutically acceptable, inert, non-toxic carriers.

5. Pharmaceutical compositions according to claim 4 for the treatment of disorders of the melatoninergic system.

6. Pharmaceutical compositions according to claim 4 for the treatment of sleep disorders, stress, anxiety, seasonal affective disorders or major depression, cardiovascular pathologies, pathologies of the digestive system, conditions of insomnia and fatigue resulting from jetlag, schizophrenia, panic attacks, melancholia, appetite disorders, obesity, insomnia, psychotic disorders, epilepsy, diabetes, Parkinson's disease, senile dementia, various disorders associated with normal or pathological ageing, migraine, memory losses, Alzheimer's disease and cerebral circulation disorders, and also in sexual dysfunctions, as ovulation inhibitors and immunomodulators and in the treatment of cancers.

## Patentansprüche

1. Kristallform V von Agomelatin der Formel (I): 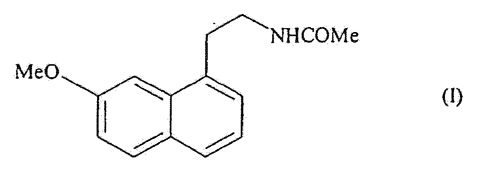 **gekennzeichnet durch** das folgende Pulverröntgenbeugungsdiagramm, gemessen mit einem Siemens-Diffraktometer D5005 (Kupfer-Antikathode) und ausgedrückt als Netzebenenabstände d, Bragg-Winkel 2-Theta und relative Intensität (ausgedrückt als Prozentsatz bezogen auf die intensivste Bande):
| **2-Theta (°)** **(exp.)** | **d (Å)** **(exp.)** | **Intensität** **(%)** |
|---|---|---|
| 9,84 | 8,979 | 17 |
| 12,40 | 7,134 | 15 |
| 13,31 | 6,646 | 19 |
| 15,14 | 5,848 | 18 |
| 15,98 | 5,543 | 18 |
| 16,62 | 5,329 | 19 |
| 17,95 | 4,939 | 100 |
| 18,88 | 4,697 | 65 |
| 20,49 | 4,332 | 24 |
| 20,99 | 4,228 | 34 |
| 23,07 | 3,852 | 39 |
| 23,44 | 3,792 | 36 |
| 24,28 | 3,663 | 58 |
| 25,10 | 3,545 | 19 |
| 26,02 | 3,422 | 15 |
| 26,82 | 3,322 | 19 |
| 27,51 | 3,239 | 16 |

2. Verfahren zur Herstellung der Kristallform V der Verbindung der Formel (I) nach Anspruch 1, **dadurch gekennzeichnet, dass** man Agomelatin in einem Mahlwerk mit hoher Energie einer mechanischen Zerkleinerung unterwirft.

3. Verfahren zur Herstellung der Kristallform V der Verbindung der Formel (I) nach Anspruch 1, **dadurch gekennzeichnet, dass** man Agomelatin bis zum vollständigen Schmelzen erhitzt, dann auf Raumtemperatur bringt und gleichzeitig eine sehr geringe Menge einer frisch hergestellten Kristallform V der Verbindung der Formel (I) zugibt und dann bis zur vollständigen Kristallisation abkühlen lässt.

4. Pharmazeutische Zubereitungen enthaltend als Wirkstoff die Kristallform V von Agomelatin nach Anspruch 1 in Kombination mit einem oder mehreren inerten, nichttoxischen und pharmazeutisch annehmbaren Hilfsstoffen.

5. Pharmazeutische Zubereitungen nach Anspruch 4 zur Behandlung von Störungen des melatoninergischen Systems.

6. Pharmazeutische Zubereitungen nach Anspruch 4 für die Behandlung von Schlafstörungen, Stress, Angst, saisonal bedingten Depressionen oder der Major-Depression, kardiovaskulären Erkrankungen, Erkrankungen des Verdauungssystems, Schlaflosigkeit und Müdigkeit als Folge von Zeitverschiebungen, Schizophrenie, Panikanfällen, Melancholie, Appetitstörungen, Fettsucht, Schlaflosigkeit, psychotischen Störungen, Epilepsie, Diabetes, der Parkinsonschen Krankheit, der senilen Demenz, verschiedenen Störungen, die mit dem normalen oder pathologischen Altern verknüpft sind, Migräne, Gedächtnisverlusten, der Alzheimerschen Krankheit, Störungen der Gehirndurchblutung sowie von sexuellen Dysfunktionen, als Ovulationsinhibitoren, Immunomodulatoren und bei der Behandlung von Krebserkrankungen.
